(19) Europäisches Patentamt

European Patent Office

Office européen des brevets

(11) Veröffentlichungsnummer: **0 372 416**
**A1**

(12) **EUROPÄISCHE PATENTANMELDUNG**

(21) Anmeldenummer: **89122201.0**

(22) Anmeldetag: **01.12.89**

(51) Int. Cl.⁵: **C07C 311/48, C07C 303/42, C01B 11/06**

(30) Priorität: **03.12.88 DE 3840815**

(43) Veröffentlichungstag der Anmeldung:
**13.06.90 Patentblatt 90/24**

(84) Benannte Vertragsstaaten:
**AT BE CH DE ES FR GB IT LI LU NL SE**

(71) Anmelder: **BEHRINGWERKE
Aktiengesellschaft
Postfach 1140
D-3550 Marburg 1(DE)**

(72) Erfinder: **Stief, Thomas, Dr.
Avda. Kansas City 28, Boque, 1. Apt. 225
E-41007 Sevilla(ES)**

(74) Vertreter: **Klein, Otto, Dr. et al
Hoechst AG Zentrale Patentabteilung
Postfach 80 03 20
D-6230 Frankfurt am Main 80(DE)**

(54) Verfahren zur Stabilisierung von Chloraminen oder Salzen von unterchloriger Säure.

(57) Es wird ein Verfahren zur Stabilisierung von Chloraminen oder Salzen von unterchloriger Säure in getrockneter Form mittels oxidationsresistenter reversibel entwässerbarer Substanzen beschrieben.

EP 0 372 416 A1

## Verfahren zur Stabilisierung von Chloraminen oder Salzen von unterchloriger Säure

Die Erfindung betrifft ein Verfahren zur Stabilisierung von Chloraminen oder Salzen von unterchloriger Säure in getrockneter Form mittels reversibel entwässerbarer oxidationsresistenter Substanzen.

Chloramine als Methionin zu Methioninsulfoxid oxidierende Agentien sind in der klinischen Chemie der Fibrinolyse von Bedeutung. Sie ermöglichen eine von anwesenden alpha-2-Antiplasmin nahezu unabhängige Messung fibrinolytischer Aktivität.

Lösungen von Chloraminen sind jedoch nur begrenzte Zeit haltbar, insbesondere wenn oxidierbare Substanzen anwesend sind. Zweckmäßig ist daher, die Chloramine in lyophilisierter Form bereitzustellen. Gebräuchliche Lyophilisationsfüllstoffe wie Mannit oder $^R$Ficoll sind jedoch ungeeignet, da sie mit Oxidantien reagieren.

Aufgabe der Erfindung ist daher, ein Verfahren zur Stabilisierung von Chloraminen zur Verfügung zu stellen.

Oxidantien vom Typ der Chloramine wie Chloramin T oder Chloramin B oder deren Salze oder Salze von HOCl in getrockneter, vorzugsweise lyophilisierter Form werden überraschenderweise durch reversibel entwässerbare oxidationsresistente Substanzen stabilisiert.

Gegenstand der Erfindung ist daher ein Verfahren zur Stabilisierung von Chloraminen oder Salzen von unterchloriger Säure in getrockneter Form, dadurch gekennzeichnet, daß eine oxidationsresistente reversibel entwässerbare Substanz zugegeben wird.

Bevorzugt sind Zucker, vorzugsweise Maltose oder Salze, vorzugsweise tri-Natriumcitrat-dihydrat oder Calciumacetat.

Die Substanzen wie Zucker, vorzugsweise Maltose, oder Salze werden in Konzentrationen von 0.1-30, vorzugsweise 1-5% (Gewichtsprozent) verwendet.

Die derart stabilisierten Oxidantien behalten in lyophilisierter Form ihre Oxidationskraft, selbst wenn die Reagenzien länger als 2 Wochen bei 37° C gelagert werden.

Die Erfindung wird weiterhin in den Ansprüchen definiert und wird in den folgenden Beispielen erläutert.


Beispiel


Stabilisierung von Chloramin durch hygroskopische Substanzen

450,7 mg Chloramin T (bzw. 341,7 mg Chloramin B) wurden zusammen mit 94,3 mg Tranexamsäure und je 600 mg Maltose Monohydrat, Calciumacetat oder tri-Natriumcitrat-dihydrat in 20 ml aqua dest. aufgenommen und die pH-Werte auf pH 10 eingestellt.

Nach vollständigem Lösen wurden die Lösungen im PAI-CAP-Test der Behringwerke als Ersatz des Oxidant-Reagenzes eingesetzt. Außerdem wurden die Lösungen in 1 ml Abfüllungen lyophilisiert und in festem (lyophilisierten) und in aufgelöstem Zustand bei 37° C gelagert und ein PAI-CAP-Test mit diesen Reagenzien durchgeführt. Als Probe wurde jeweils Tris-Puffer bzw. Standard S1 aus dem PAI-Testkit der Behringwerke eingesetzt. Die Ergebnisse sind in der folgenden Tabelle dargestellt.

2

Tabelle

| | Chloramin T gem.Plasminakt.*(A/5min) x 1000 | | | Chloramin B |
| --- | --- | --- | --- | --- |
| | Puffer | Standard S1 | Abfall der Akt.in St. S1 gegenü. Puffer(%) | |
| **Maltose** | | | | |
| sofort | 1.298 | 1.210 | 7 | 10 |
| nach 1 Wo. | 1.271 | 1.076 | 15 | 23 |
| nach 2 Wo. | 1.078 | 0.909 | 16 | 25 |
| **Calciumacetat** | | | | |
| sofort | 1.262 | 1.156 | 8 | 15 |
| nach 1 Wo. | 1.212 | 1.051 | 13 | 23 |
| nach 2 Wo. | 1.160 | 1.002 | 14 | 22 |
| **Tri-Natriumcitrat-Dihydrat** | | | | |
| sofort | 1.363 | 1.205 | 12 | 15 |
| nach 1 Wo. | 1.304 | 1.036 | 21 | 25 |
| nach 2 Wo. | 1.190 | 0.971 | 18 | 20 |

* Mittelwerte aus Doppelbestimmungen

Maltose, Calciumacetat oder tri-Natriumcitrat-dihydrat erweisen sich als nahezu oxidationsinert gegenüber Chloraminen und stabilisieren diese.

**Ansprüche**

1. Verfahren zur Stabilisierung von Chloraminen oder Salzen von unterchloriger Säure in getrockneter Form, dadurch gekennzeichnet, daß eine oxidationsresistente reversibel entwässerbare Substanz zugegeben wird.

2. Verfahren nach Anspruch 1, dadurch gekennzeichnet, daß die Substanz Maltose, Calciumacetat oder tri-Natriumcitrat-dihydrat ist.

3. Verfahren nach Anspruch 1, dadurch gekennzeichnet, daß die getrocknete Form durch Gefriertrocknen erhalten wird.

4. Verfahren nach mindestens einem der Ansprüche 1 bis 3, dadurch gekennzeichnet, daß die Substanzen in Konzentrationen von 0.3-30 Gewichtsprozent verwendet werden.

Europäisches
Patentamt

**EUROPÄISCHER RECHERCHENBERICHT**

Nummer der Anmeldung

EP 89 12 2201

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | CHEMICAL ABSTRACTS, Band 99, 1983, Seite 123, Zusammenfassung 107499, Columbus, Ohio, US; & ES-A-510 173 --- | | C 07 C 311/48<br>C 07 C 303/42<br>C 01 B 11/06 |
| A | US-A-1 784 286 (R.R. HERSHMAN) --- | | |
| A | DE-C- 402 983 (CHEMISCHE FABRIK VON HEYDEN) --- | | |
| A | FR-A-2 087 248 (DU PONT)<br>* Ansprüche 1,9 * --- | | |
| A | US-A-4 124 734 (D.S. ALTERMAN) ----- | | |
| | | | **RECHERCHIERTE SACHGEBIETE (Int. Cl.5)**<br><br>C 07 C 311/00<br>C 01 B 11/00 |

Der vorliegende Recherchenbericht wurde für alle Patentansprüche erstellt

| Recherchenort | Abschlußdatum der Recherche | Prüfer |
|---|---|---|
| DEN HAAG | 12-02-1990 | ZAROKOSTAS K. |